# EUROPEAN PATENT SPECIFICATION

(11) **EP 0 876 155 B1**
(45) Date of publication and mention of the grant of the patent: **07.07.2004**
(21) Application number: 97904797.4
(22) Date of filing: 09.01.1997
(51) Int. Cl.: A61K 38/48, A61K 47/00

(54) **HIGHLY CONCENTRATED, LYOPHILIZED AND LIQUID FACTOR IX FORMULATIONS**
HOCHKONZENTRIERTE, LYOPHILISIERTE UND FLÜSSIGE FAKTOR-IX FORMULIERUNGEN
FORMULATIONS DE FACTEUR IX TRES CONCENTREES, LYOPHILISEES ET LIQUIDES

(30) Priority: 25.01.1996 US 591332
(43) Date of publication of application: 11.11.1998
(73) Proprietor: Genetics Institute, LLC, Cambridge, MA 02140 (US)
(72) Inventor: WEBB, Chandra, Pelham, NH 03076 (US); BUSH, Lawrence, Tewksbury, MA 01876 (US); SCHAUB, Robert, G., Pelham, NH 03076 (US)
(74) Representative: Ingham, Stephen H.
(86) International application number: PCT/US1997/000747
(87) International publication number: WO 1997/026909

(56) References cited:
- EP-A- 0 317 376
- EP-A- 0 430 200
- WO-A-91/10439
- WO-A-95/26750
- WO-A-95/28954

## Description

### FIELD OF INVENTION

The present invention relates generally to novel formulations comprising factor IX, including both highly concentrated, lyophilized, and liquid formulations comprising factor IX suitable for administration via various routes including for example routes such as *intravenous*, *subcutaneous*, *intramuscular* and *intradermal*.

### BACKGROUND OF THE INVENTION

A variety of factors involved in the blood clotting process have been identified, including factor IX, a plasma glycoprotein. A deficiency of factor IX characterizes a type of hemophilia (type B). Treatment of this disease has traditionally involved *intra venous* infusion of human plasma-derived protein concentrates of factor IX. Infusion of blood concentrates involves the risk of transmission of various infectious agents, such as viral hepatitis and HIV, or thromboembolic factors. An alternative method of producing factor IX, by recombinant DNA techniques, has been described in USPN 4,770,999, Kaufman *et al*., September 13, 1988. The cDNA coding for human factor IX has been isolated, characterized, and cloned into expression vectors. *See,* for example, Choo *et al*., Nature 299.178-180 (1982); Fair *et al*., Blood 64:194-204 (1984): and Kurachi *et al*., Proc. Nat. Acad. Sci., U.S.A. *79*:6461-6464 (1982). Thus, through advances in recombinant DNA technology, it has been possible to produce factor IX protein.

It is desirable to have both bulk and finished forms of factor IX, suitable for both storage and for delivery. Typically, a purification process for a protein results in concentrating the protein. This concentrated protein, also known as bulk protein, may be in a formulation buffer. Bulk protein. typically at a concentration of about 2 to at least 20 mg/mL, can then be shipped frozen to a fill/finish facility where it is adjusted to an appropriate dosage concentration and placed into dosage vials or some device suitable for administration, e.g. a pre-fillable syringe. Ideally, the drug product is left in the liquid state and stored and administered as a liquid. Alternatively, the drug product is lyophilized, *i*.*e*., freeze-dried. Ideally lyophilized drug product has sufficient stability to be kept in long-term storage, *i*.*e*., greater than six months: lyophilized drug product is reconstituted at a later time by adding a suitable administration diluent just prior to patient use.

The decision to either maintain the finished drug product as a liquid or to freeze-dry it is usually based on the stability of the protein drug in those forms. Protein stability can be affected *inter alia* by such factors as ionic strength, pH, temperature, repeated cycles of freeze/thaw, and exposures to shear forces. Active protein may be lost as a result of physical instabilities, including denaturation and aggregation (both soluble and insoluble aggregate formation), as well as chemical instabilities, including, for example, hydrolysis, deamidation, and oxidation, to name just a few. For a general review of stability of protein pharmaceuticals, see, for example, Manning, *et al*., Pharmaceutical Research *6*:903-918 (1989).

While the possible occurrence of protein instabilities is widely appreciated, it is impossible to predict particular instability problems of a particular protein. Any of these instabilities can result in the formation of a protein, protein by-product, or derivative having lowered activity, increased toxicity, and/or increased immunogenicity. Indeed, protein precipitation may lead to thrombosis, non-homogeneity of dosage form and amount, as well as clogged syringes. Also, specific to factor IX, there are several post-translational modifications (for example. the gamma carboxylation of certain glutamic acid residues in the N-terminus and the addition of carbohydrate) all of which provide potential sites that may be susceptible to modification upon storage. Thus, the safety and efficacy of any pharmaceutical formulation of a protein is directly related to its stability. Maintaining that stability in a liquid dosage form is generally different from a lyophilized dosage form because of greatly increased potential for molecular motion and therefore increased probability of molecular interactions. Maintaining stability in a highly concentrated form is also different because of the propensity for aggregate formation at high protein concentrations.

When developing a liquid formulation, many factors are taken into consideration. Short-term. *i*.*e*., less than six months, liquid stability generally depends on avoiding gross structural changes, such as denaturation and aggregation. These processes are described in the literature for a number of proteins, and many examples of stabilizing agents exist ("Strategies to Suppress Aggregation of Recombinant Keratinocyte Growth Factor during Liquid Formulation Development", B.L. Chen *et al*., J. Pharm. Sci. 83(12):1657-1661, (1994); "Formulation Design of Acidic Fibroblast Growth Factor", P.K. Tsai *et al*., Pharm. Res. 10(5):649-659 (1993); "The Stabilization of Beta-Lactoglobulin by Glycine and NaCl", Tsutomu Arakawa, Biopolymers 28:1397-1401 (1989); "Structural stability of lipase from wheat germ". A.N. Rajeshwara and V. Prakash, Internat. J. of Peptide & Prot. Res. 44:435-440 (1994); "Thermal Stability of Human Immunoglobulins with Sorbitol", M. Gonzalez *et al*., Vox Sang 68:1-4 (1995)). It is well known that an agent effective at stabilizing one protein structural changes, developing a liquid formulation for long-term stability (greater than six months, for example) depends on further stabilizing the protein from types of degradation specific to that protein. More specific types of degradation may include, for example, disulfide bond scrambling, oxidation of oligosaccharides and/or certain residues, deamidation, cyclization, and the like. Although it is not always possible to pinpoint the individual degradation species, assays are developed to monitor subtle changes so as to monitor the ability of specific excipients to uniquely stabilize the protein of interest.

In addition to stability considerations, one generally selects excipients which will meet with the approval of various world-wide medical regulatory agencies. It is highly desirable that the formulation be approximately isotonic and that the pH of the formulation be in a physiologically suitable range upon injection/infusion, otherwise pain and discomfort for the patient may result. The choice and amount of buffer used is important to achieve the desired pH range. The choice and amount of agents used to modify tonicity is important to assure ease of administration.

Traditionally, large labile proteins, such as factor IX, are administered *intravenous*, either prophylactically or in response to bleeding episodes. Given *intravenous*, the protein is directly available in the blood stream. Unfortunately, there can be side effects associated with repeated injections, including occlusion and/or fibrin formation, especially in the elderly. Moreover, where the patient's veins are particularly small, *e*.*g*., in small children, it can be difficult to achieve the requisite therapeutic dose.

EP 0317376 discloses a chromatographic method of separating a fraction of human plasma containing factor IX at a concentration of 25 U/ml. The addition of arginine to the elution buffer in order to stabilize the factor IX is described.

Currently, there are no highly concentrated factor IX formulations commercially available. The only two commercially available (in the US), carrier- protein-free. plasma-derived factor IX formulations, are freeze-dried products which are reconstituted for use, and are limited to low factor IX concentrations, *e*.*g*., about 100 U/mL or less than 1 mg/mL. Such low concentrations are primarily indicated for *intravenous* administration and not intended for subcutaneous, *intramuscular,* or *intradermal* use. Alpha Therapeutic Corporation provides lyophilized AlphaNine® SD, comprising heparin, dextrose, polysorbate 80, and tri(n-butyl) phosphate. This preparation is meant to be stored at temperatures between 2° and 8°C. Heparin is to be avoided as it is an anti-coagulant and tri(n-butyl) phosphate is irritating to mucous membranes; thus, this formulation is less than ideal. Armour Pharmaceutical Company provides lyophilized Mononine®, comprising histidine, sodium chloride and mannitol, is similarly meant to be stored at 2° to 8°C. The package insert recommends not storing this formulation for greater than one month at room temperature. There are no liquid nor any highly concentrated factor IX products currently commercially available. Schwinn, PCT/EP90/02238, discloses factor IX, 0.9 M saccharose, 0.5 M lysine, and 0.003 M calcium chloride, stored at 4-8°C, stable for only a period of weeks and therefore, unsuitable for commercial production; this formulation is, unfortunately hypertonic and the pH is outside the range for comfortable administration, and therefore unsuitable for injection.

Easier to handle for the patient are administration forms such as *subcutaneous*, *intramuscular, or intradermal*. *Subcutaneous* administration of factor IX is described in Berrettini, Am. J. Hematol. 47:61 (1994) and in WO 93/O7890, BTG, Brownlee (published 29 April 1993). In Berrettini, an Immuno product was used: Immunine™; factor IX, heparin, sodium citrate. sodium chloride, and antithrombin III at a concentration of 118 U/mg. The product was reportedly poorly and slowly transported into the circulation and the authors concluded that *subcutaneous* administration was not reliable for treating or preventing bleeding in hemophilia B patients and that even more concentrated forms would be unacceptable in terms of clinical efficacy. Brownlee, *supra*, discloses a Mononine™ factor IX formulation at a concentration of 10-500 U/mL. Only low circulating levels were obtained and at page 9, it is noted that after four hours large clots had formed under the skin at each site where the factor IX had been injected resulting in severe bruising. Such can be observed when using a product that is impure.

In hemophilia B dogs (Brinkhous, *et al*., FASEB 7:117 (1993)) and in one hemophilia B patient (Liles, *et al*., Thromb. Haemost. 73:1986a (1995)), **plasma** derived factor IX (pFIX) was administered *subcutaneously* (at doses of 15-47 U/kg in dogs and at a dose of 30 U/kg in the patient). This resulted in plasma factor IX in dogs which was dose dependent and ranged from 0.8 to 7.6 %, with *intramuscular* giving higher levels. In the hemophilia patient, plasma factor IX activity reached only 1% within six hours; this level of activity persisted for 36 hours; the low concentration of **plasma-derived** factor IX required high volume injections at multiple (10) sites.

One of the major problems associated with formulating a suitable *subcutaneous* formulation is achieving a high enough concentration of the protein, without causing aggregate formation of the protein and without simultaneously concentrating impurities in the preparation. Both aggregate formation and impurities lead to increased immunogenicity. With currently available products, to give an appropriate dose of factor IX *subcutaneously* requires the use of multiple injection sites. This causes great discomfort and inconvenience to the patient. To practically deliver factor IX *subcutaneously,* it is necessary to concentrate factor IX to at least 1.000 U/mL or greater and provide it in a stable, non-aggregating dosage form. Such a concentrated form is currently unavailable.

Ideally, formulations should provide for factor IX stability for greater than one year and for compatibility over a wide range of protein concentration (0.1 mg/mL to greater than 160 mg/mL, *i*.*e*., 20 U/mL to greater than 56,000 U/mL for example). This allows for flexibility in methods of administration which may require high protein concentration, *e*.*g*., *subcutaneous, intradermal*, or *intramuscular* administration, or those which may utilize low protein concentration, *e.g. intravenous* administration. Generally, more highly concentrated forms allow for the administration of lower volumes which is highly desirable from the patients' point of view. Liquid formulations can have many advantages over freeze-dried products with regard to ease of administration and use. Accordingly, there continues to exist a need in the art for methods for improving factor IX protein stability, increasing the concentration, maintaining activity levels, and providing stable liquid formulations suitable for prolonged storage for greater than one year at 2 to 8°C.

### BRIEF SUMMARY OF THE INVENTION

The present invention relates to compositions and highly concentrated, lyophilized, and liquid preparations of factor IX useful as bulk protein or useful for administration. The invention provides a composition comprising highly concentrated factor IX and arginine, a buffering agent, and sucrose or mannitol, wherein the factor IX concentration is about 2 to about 160 mg/mL. These compositions, either frozen or liquid, are stable for at least six months, and preferably up to 36 and 60 months; and can be stored at temperatures ranging from -100°C to 40°C, from -80°C to O°C, and from -20°C to 10°C. The compositions comprise factor IX, tonicity modifiers, cryoprotectants, and a buffering agent and/or other excipients which further stabilize factor IX. The factor IX concentration ranges from about 2 to about 160 mg/mL (160 mg/mL being equivalent to at least 56,000 U/mL), and 2 to 10 mg/mL (2500 U/mL) preferred, the most preferred range depending upon the route of administration. Tonicity modifiers include, but are not limited to, salts, sugars, polyols, and amino acids. Suitable amino acids include arginine, glycine and histidine at a concentration of about 10 to 500 mM, with about 10 to 300 mM and about 10 to 200 mM preferred. Arginine concentration is preferably about 130 to 160 mM, about 130 to 235 mM or about 60 to 70 mM. Most preferably arginine concentration is about 160 mM. Cryoprotectant polyols, mannitol and sucrose, range in concentration from about 1 to 400 mM, with about 5 to 200 mM and 20 to 100 mM preferred. Sucrose concentration is preferably about 3 to 60 mM. Optionally, the compositions may also contain a surfactant or detergent, such as polysorbate (*e*.*g*. Tween) or polyethyleneglycol (PEG), which may also serve as a cryoprotectant during freezing. The surfactant ranges from about 0.005 to 1 %, with about 0.005 to 0.1 % and about 0.005 to 0.02 % preferred. The compositions contain an appropriate buffering agent to maintain a physiologically suitable pH, *e*.*g*., in the range of about 5.8 to 8.0 with about 6.2 to 7.2 and about 6.5 to 7.0 being preferred. Buffering agents preferably include histidine, sodium citrate, potassium citrate. maleic acid, ammonium acetate, Tris, sodium phosphate, potassium phosphate, and diethanolamine, with sodium/potassium citrate preferred, with a preferred pH of about 6.5 to 7.5, and a concentration range of about 1-100 mM, with 5 to 50 mM and 10 to 25 mM preferred. Preferred citrate concentration is about 1 to 40 mM, most preferred about 15 mM. Optionally small amounts of a chelator such as EDTA are included, at a concentration of 0.05 to 50 mM. or 0.05 to 10 mM, or 0.1 to 5 mM, with about 1 to 5 mM preferred.

Another aspect of the present invention provides formulations of factor IX suitable for administration in a final dosage form, for example, via *intravenous*, *subcutaneous, intradermal*, or *intramuscular* routes of administration. Typically, large quantities of bulk drug are frozen and can be shipped, if necessary, to a manufacturing site where the bulk drug is filled into small vials; if desired, the final dosage form is a diluted, pH-adjusted form; bulk drug typically comprises a higher protein concentration than finished drug and does not need to be isotonic. The finished drug compositions comprise factor IX, tonicity modifiers, cryoprotectants and a buffering agent and/or other excipients which further stabilize factor IX, as described, *supra*. The finished drug formulations are stable for at least six months and preferably up to 36 and 60 months; and can be stored at temperatures ranging from -100°C to 40°C, from -20°C to 37°C and from 2°C to 8°C. The concentrations of the excipients provide a combined osmolality of about 250 to 420 milliosmolal. Preferred formulations include factor IX concentrations ranging from about 2 to greater than 160 mg/mL (to greater than 56,000 U/mL); with sodium citrate as a buffering agent; some combination of mannitol, sucrose, arginine, and glycine as cryoprotectants and tonicity modifiers; and optionally small amounts of a chelator, such as EDTA (*ca*. 1 to 5 mM) and/or small amounts of polysorbate (0.005% to 0.02%).

The compositions of the present invention may comprise an arginine concentration of about 130 to 160 mM, a sucrose concentration of about 0 to 60 mM and a surfactant concentration of about 0 to 0.005%; alternatively said arginine concentration is about 60 to 70 mM, said buffering agent is about 0 to 20 mM sucrose and 165 mM mannitol, and said surfactant concentration is 0 to 0.005%. In a further composition arginine concentration is about 160 mM, said sucrose concentration is about 0 mM, said citrate concentration is about 15 mM and said surfactant concentration is about 0%; or said arginine concentration is about 70mM, said mannitol concentration is about 165 mM, said citrate concentration is about 15 mM, and said surfactant or chelating agent is 0%.

Further compositions comprise highly concentrated factor IX and arginine, a buffering agent, and sucrose or mannitol in formulations comprising;
about:
   2 to 160 mg/mL factor IX,
   130 to 235 mM arginine, and
   7.5 to 40 mM citrate.
about:
   2 to 160 mg/mL factor IX,
   66 to 90 mM arginine,
   110 to 165 mM mannitol, and
   7.5 to 40 mM citrate.
about:
   2 to 160 mg/mL factor IX,
   70 mM arginine,
   165 mM mannitol, and
   15 mM citrate.
about:
   2 to 160 mg/mL factor IX,
   160 mM arginine, and
   15 mM citrate.
Where the surfactant is polysorbate an alternative composition comprises about:
2 to 160 mg/mL factor IX,
130 to 235 mM arginine,
0 to 60 mM sucrose,
0 to 0.005% polysorbate, and
7.5 to 40 mM citrate.
The invention also provides for the use of a composition as descried above for the preparation of a pharmaceutical composition for increasing factor IX concentration preferably said pharmaceutical composition is designed to be administered both *intravenously* and subcutaneously.

Also provided are methods of administration of highly concentrated factor IX using both *intravenous* and *subcutaneous* routes, e. g., an *intravenous* dose followed by a *subcutaneous* dose.

### DETAILED DESCRIPTION OF THE INVENTION

As used herein, factor IX includes both plasma derived and recombinantly or synthetically produced. Factor IX concentration is conveniently expressed as mg/mL or as U/mL, with 1 mg usually representing > 150 U ± 100 U or more. One Unit of activity is defined as the amount of factor IX clotting activity in one milliliter of normal human plasma. The specific activity is the ratio of clotting activity concentration to protein concentration, expressed as U/mg of protein. Patients with hemophilia generally have from < 1 to 25 % of the factor IX clotting factor as is found in normal human plasma.

As used herein, amounts specified are understood to be ± about 10%, e.g., about 50 mM includes 50 mM ± 5 mM; *e*.*g*., 4% includes 4% *±* 0.4%, *etc*.

As used herein, the term "tonicity modifier" includes agents which contribute to the osmolality of the solution. Examples of tonicity modifiers include, but are not limited to, amino acids such as arginine, histidine. and glycine, salts such as sodium chloride. potassium chloride, and sodium citrate, and saccharides such as sucrose, glucose, and mannitol, and the like.

The term "cryoprotectant" generally includes agents which provide stability to the protein from freezing-induced stresses; however, cryoprotectants may also provide general stability, for example for bulk drug formulations during storage from non-freezing-induced stresses. Exemplary cryoprotectants include polyols, and saccharides such as mannitol and sucrose, as well as surfactants such as polysorbate, or polyethyleneglycol, and the like. While preferred concentrations of cryoprotectant range from about 0.2 to 4% (weight/volume), relatively high concentrations, for example greater than 5%, are also suitable; the levels used are limited only by those customarily used in clinical practice. The upper concentration limits for bulk drug may be higher than for finished dosage, *e*.*g*., greater than 5%. "Surfactants" generally include those agents which protect the protein from air/solution interface induced stresses and solution/surface induced stresses (e.g., resulting in protein aggregation), and may include detergents such as polysorbate-80 (Tween), for example, about 0.005 to 1 % (volume/volume), or polyethyleneglycol (PEG), such as PEG8000, for example. Optionally, relatively high concentrations, *e*.*g*., up to 0.5%, are suitable for maintaining protein stability; however, the levels used in actual practice are customarily limited by clinical practice.

The term "buffering agent" encompasses those agents which maintain the solution pH in an acceptable range and may include histidine, phosphate (sodium or potassium), citrate (sodium or potassium), maleic acid, ammonium acetate, tris (tris (hydroxymethyl) aminomethane), diethanolamine, and the like. The upper concentration limits may be higher for bulk protein than for finished dosage protein forms as is readily appreciated by one skilled in the art. For example, while buffer concentrations can range from several millimolar up to the upper limit of their solubility, *e*.*g*., citrate, could be as high as 200 mM, one skilled in the art would also take into consideration both achieving and maintaining a physiologically appropriate concentration. Percentages are weight/volume when referring to solids dissolved in solution and volume/volume when referring to liquids mixed into solutions. For example, for sucrose, it is dry weight sucrose/volume of solution and for Tween, it is the volume of 100% stock/volume of solution. The term "isotonic with serum," 300 ± 50 milliosmolal, is meant to be a measure of osmolality of the solution prior to administration. Maintaining physiological osmolality is important for the dosage formulations to be injectable without prior dilution. However, for bulk formulations, much higher osmolalities can be effectively utilized as long as the solution is made isotonic prior to use. The term "excipients" includes pharmaceutically acceptable reagents to provide appropriate tonicity, cryoprotection of the protein, maintenance of pH, and proper conformation of the protein during storage so that substantial retention of biological activity and protein stability is maintained.

### Effect of Calcium Ions

The preparation of recombinant factor IX has been described in USPN 4,770,999, Kaufman, *et al*. One suitable purification method is that described in Hrinda, *et al*., Preclinical Studies of a Monoclonal Antibody - Purified factor IX, Mononine™ Seminars in Hematology, 28(3):6 (July 1991). Other methods of preparation include the use of conformation-specific monoclonal antibodies as described by Tharakan, *et al*., "Physical and biochemical properties of five commercial resins for immunoaffinity purification of factor IX. " Journal of Chromatography 595:103-111 (1992); and by Liebman, *et al*., "Inununoaffinity purification of factor IX (Christmas factor) by using conformation-specific antibodies directed against the factor IX-metal complex." Proc. Nat. Acad. Sci., USA 82:3879-3883 (1985); as well as conventional chromatographic procedures, for example, as described by Hashimoto, *et al*., "A Method for Systematic Purification from Bovine Plasma of Six Vitamin K-Dependent Coagulation Factors: Prothrombin, Factor X, Factor IX, Protein C, and Protein Z." J. Biochem. 97:1347-1355 (1985), and Bajaj, P. *et al*. Prep. Biochem. *11*:397 (1981). "Large-scale preparation and biochemical characterization of a new high purity factor IX concentrate prepared by metal chelate affinity chromatography", P.A. Feldman et. al., Blood Coagulation and Fibrinolysis 5:939-948 (1994). Yet another method of purification is described in USSN 08/472.823, filed June 7, 1995; and incorporated herein by reference.

A well characterized property of factor IX is its ability to bind Ca²⁺ ions. Structural studies indicate that Ca²⁺ binding may confer a more stable structure. reducing the probability of molecular motion ("Structure of the Metal-free γ-Carboxyglutamic Acid-rich Membrane Binding Region of Factor IX by Two-dimensional NMR Spectroscopy", S. J. Freedman, B. C. Furie. B. Furie, and J. D. Baleja, J. Biol. Chem. 270(14):7980-7987 (1995); "Structure of the Calcium Ion-Bound γ-Carboxyglutamic Acid-Rich Domain of Factor IX." S. J. Freedman, B. C. Furie. B. Furie, and J. D. Baleja, Biochemistry 34:12126-12137 (1994); "The Structure of a Ca²⁺-Binding Epidermal Growth Factor-like Domain: Its Role in Protein-Protein Interactions", S. Rao. P. Handford, M. Mayhew, V. Knott, G. Brownlee, and D. Stuart, Cell 82:131-141 (1995); "Structure of Ca²⁺ Prothrombin Fragment 1 Including the Conformation of the Gla Domain", M. Soriano-Garcia, C.H. Park, A. Tulinsky, K. G. Ravichandran, and E. Skrzypczak-Jankun. Biochem. 28:6805-6810 (1989)). Presumably, less mobility accords a lower probability of molecular interaction, thereby reducing the probability of degrading processes. Surprisingly, this turns out not to be the case.

Samples are prepared in the formulations set forth in Table I below, at a recombinant factor IX protein concentration of ∼0.5 mg/ml (100 U/ml) and an osmolality of 300 ± 50 milliosmolal. All samples contain a recombinant form of factor IX. To examine the potential utility of Ca²⁺ as a stabilizing agent, a set of samples was prepared in the formulations listed in Table 1. The formulation of sample A is the formulation used for commercially available plasma-derived lyophilized factor IX (Mononine™). All samples contain a recombinant form of factor IX.

**Table 1**

| **Sample Formulations** | | | | |
|---|---|---|---|---|
| Sample | pH | Buffer (10 mM) | Salt (Tonicity Modifier) | Other Excipient |
| A | 7.0 | histidine | 0.066 M NaCl (0.385%) | 165 mM mannitol |
| B | 7.0 | histidine | 260 mM glycine | 29 mM sucrose |
| C | 7.0 | histidine | 250 mM glycine, 5 mM Ca²⁺ | 29 mM sucrose |
| D | 7.5 | tris | 260 mM glycine | 29 mM sucrose |
| E | 7.5 | tris | 250 mM glycine, 5 mM Ca²⁺ | 29 mM sucrose |
| F | 7 .5 | diethanolamine | 260 mM glycine | 29 mM sucrose |
| G | 7.5 | diethanolamine | 250 mM glycine, 5 mM Ca²⁺ | 29 mM sucrose |

Samples of factor IX in each formulation were stored at 4°C for 2.5 months. Samples were assayed for protein concentration and clotting activity. Factor IX activity is determined according to the method of Pittman, D., *et al*., Blood 79:389-397 (1992) utilizing factor IX-deficient blood. The ratio of clotting activity to protein concentration, the specific activity, expressed as Units/mg of protein, is given in Table 2. An acceptable specific activity is one that is generally no more than 20% higher than the starting specific activity because an unusually high specific activity may indicate an activation-like event, which may have thrombotic implications.

**Table 2**

| **Factor IX Specific Activity** | | |
|---|---|---|
| Sample | time zero | 2.5 months |
| A | 219.9 | 161.3 |
| B | 191.8 | 153.2 |
| C | 239.4 | 964.1 |
| D | 209.3 | 135.8 |
| E | 212.1 | 1956.9 |
| F | 190.1 | 123.5 |
| G | 217.3 | 2570.8 |

The samples containing calcium, *i*.*e*., samples C, E, and G, have higher specific activities after 2.5 months of storage. This is due to the inclusion of Ca²⁺ and indicates that the factor IX has undergone a conversion to an activated-like molecule. Activated Factor IX is Factor IX that has been cleaved at residues R¹⁴⁵ - A¹⁴⁶ and R¹⁸⁰ - V¹⁸¹ and is then able to catalyze clotting. Normally, Factor IX circulates as intact protein and is not converted to its activated form unless there is initiation of the clotting cascade. Injecting someone with activated rhFIX could have thrombotic implications. Therefore inclusion of Ca2⁺ at a concentration of 5 mM is destabilizing and is to be avoided.

### Effects of Buffer Choice on HMW formation

The average specific activity after eight months of 40°C storage of samples formulated in buffer/excipient combinations similar to and including those in Table 1, but without calcium, at pH 7.0 is 112.5 ± 10.5 U/mg, but at pH 7.5 is only 84.0 ± 22.1 U/mg, indicating subtle shifts in pH are significant for maintaining long-term factor IX stability.

Factor IX is prepared in a set of isotonic experimental formulations as summarized in Table 3, including several different excipient combinations for each buffering agent and some including less than 5 mM EDTA. Factor IX concentrations are approximately 1 mg/mL (average 161 U/mL). Samples are assayed for the amount of high molecular weight material (HMW) present and for clotting activity. The formation of significant (> 3 %) amounts of HMW is undesirable and as indicative of physical degradation of factor IX with possible impact on product safety and efficacy. HMW is especially undesirable for *subcutaneous* administration as aggregated proteins are more immunogenic when given *subcutaneously*. Morein, B. and K. Simons, Vaccine 3:83. Subunit vaccines against enveloped viruses: virosomes, micelles, and other protein complexes (1985); and Antibodies: A laboratory manual, (page 100), E. Harlow and D. Lane, Cold Spring Harbor Laboratory, 1988.

**Table 3**

| **Sample Formulation** | |
|---|---|
| **Buffering Agent (10-15 mM)** | **Excipients** |
| **Phosphate** (either sodium or potassium phosphate, pH 7.0) | arginine-HCl, sodium chloride, glycine, sucrose, mannitol, glucose |
| **Citrate** (sodium, pH 6.0-6.5) | sorbitol, glucose, glycine, sucrose, arginine-HCl |
| Ammonium Acetate (pH 6.5-7.0) | mannose, mannitol, sodium chloride, arginine-HCl |
| **Maleic Acid** (pH 6.5) | glycine, mannose |

Table 4 shows the effects of the different buffering agents on HMW generation as measured by size exclusion chromatography (SEC-HPLC). Samples were stored at 3 six weeks. Table 4 gives the average increase expressed as (HMW/total protein x 100%) at six weeks minus that at time zero.

**Table 4**

| **Percent Increase HMW Generation** | |
|---|---|
| Buffering Agent | Avg. Increase (% of total) |
| Phosphate: | 4.21 |
| Citrate: | 0.80 |
| Ammonium Acetate: | 3.24 |
| Maleic Acid: | 1.67 |

The citrate buffered samples had, on average, the smallest amount of HMW generated, regardless of the other excipients included. An appropriate buffer does not allow greater than a 2 % increase.

Aggregates are commonly known to be more immunogenic than monomeric proteins and are generally not acceptable for an *intravenous* formulation. However, aggregates are even more undesirable for a *subcutaneous, intradermal* or *intramuscular* formulation, because these routes of administration are more likely to generate an immune response.

All samples are stored further for six months at 4°C and assayed for clotting activity. The average amount of activity remaining for samples containing the various saccharides varied greatly; sucrose-containing samples maintained an average 71 % of the starting activity, mannitol 53%, glucose 52%, and mannose only 27%. Surprisingly, not all saccharides are equally effective at maintaining factor IX activity, despite the addition of other excipients.

The following examples illustrate practice of the invention. These examples are for illustrative purposes only and are not intended in any way to limit the scope of the invention claimed. Example 1 illustrates the use of the invention for higher concentrations of factor IX. Example 2 illustrates the complexity of excipient interactions in stabilizing factor IX. Example 3 describes factor IX in various formulations relating to freeze/thaw stability. Example 4 describes the effects of long term storage, and Examples 5 and 6 illustrate highly concentrated forms and their use.

### Example 1: Stability at high concentration

Another set of formulations is prepared comprising higher concentrations of factor IX; samples are prepared in the formulations listed in Table 5 at a concentration of 8 mg/mL (2000 U/mL). All contain 15 mM sodium citrate and are buffered at pH 6.8, without surfactant. BG4 is slightly hypertonic, the rest are isotonic.

**Table 5**

| **Sample Formulations** | |
|---|---|
| BG1: | 2% sucrose, 2% arginine-HCl, 1 mM EDTA |
| BG2: | 4% sucrose, 1% glycine, 1 mM EDTA |
| BG3: | 1.5% arginine-HCl, 1% glycine |
| BG4: | 5% atginine-HCl, 1 mM EDTA |
| BG5: | 4% sucrose. 1% glycine |

Samples are stored in both glass vials and glass prefillable syringes for eight, ten and thirteen months at 4°C to determine whether the amount of air/solution interface or siliconized stopper/solution interface would impact the stability of the product. At all three time points no significant differences are seen by any stability assays between the vials and syringes. The results of several analytical methods are shown in Table 6. "Recovery of Activity" refers to the amount of clotting activity remaining in the sample expressed as a percentage of the amount of clotting activity present at "time zero", the start of the study. "HMW" is described, *supra*. "SDS-PAGE" is polyacrylamide gel electrophoresis; gels are scanned and bands quantified. Reversed phase HPLC is used to evaluate product heterogeneity and changes in peak ratios may indicate changes in the product, for example, oxidation of oligosaccharides.

**Table 6**

| Sample | Recovery ot Activity as % of control | | HMW, as determined by SEC-HPLC | | % full-length FIX. by SDS-PAGE | | Reversed Phase HPLC ratio assay | |
|---|---|---|---|---|---|---|---|---|
| | 8 mos. | 13 mos. | 8 mos. | 13 mos. | 8 mos. | 10 mos. | 8 mos. | 13 mos. |
| BG-1 | 90% | 92% | 0.31 | 0.37 | 98.2 | 98.5 | 0.33 | 0.35 |
| BG-2 | 78% | 82% | 0.33 | 0.32 | 98.0 | 98.4 | 0.32 | 0.37 |
| BG-3 | 85% | 82% | 0.35 | 0.36 | 98.2 | 98.5 | 0.34 | 0.37 |
| BG-4 | 83% | 81 % | 0.25 | 0.28 | 98.6 | 99.1 | 0.33 | 0.36 |
| BG-5 | 74% | 81% | 0.40 | 0.35 | 98.3 | 98.6 | 0.32 | 0.34 |

Even at this higher concentration of factor IX (8 mg/mL; 2000 U/mL) these formulations are effective. The increase in observed activity for certain samples at 13 months, relative to the control, is within the variability of the assay.

### Example 2: Excipient Interactions

Another set of factor IX formulations, all containing citrate, is prepared as summarized in Table 7. All formulations are isotonic, contain factor IX at concentrations of 1 to 2 mg/mL (average 208 to 481 U/mL), use 10 mM to 15 mM sodium citrate as the pH buffering agent, and are adjusted to pH 6.8.

**Table 7**

| **Sample Formulations** | |
|---|---|
| **Major Excipient** (range of concentration, wt/vol %) | **Used in combination with:** |
| mannitol (55 - 275 mM, 1-5%) | arginine-HCl, EDTA, glycine, Tween-80, sucrose, NaCl, KCl |
| arginine-HCl (47 - 237 mM, 1-5%) | mannitol, EDTA, sucrose, glycine, Tween-80, glucose |
| glycine (66 - 306 mM, 0.5-2.3%) | mannitol, arginine-HCl, glucose, Tween-80, EDTA |
| sucrose (29 - 234 mM,1-8%) | mannitol, arginine-HCl, glycine, NaCl, EDTA, Tween-80 |
| glucose (55 - 278 mM, 1-5%) | arginine-HCl, glycine, NaCl, KCl, EDTA |
| NaCl (100 mM, 0.58%) | sucrose, glucose, mannitol, EDTA |
| KCI (100 mM, 0.75%) | glucose, mannitol |

Samples are stored at 4°C and assayed at several points in time. After eight months of 4°C storage, nine samples maintain ∼ 100% of the clotting activity of the starting material. The formulations of these nine are shown in Table 8 (all include 15 mM sodium citrate, are pH 6.8, and isotonic).

**Table 8**

| | |
|---|---|
| 1 | 4% sucrose, 1.4% glycine, 0.005% Tween-80 |
| 2 | 1% mannitol, 2 % arginine-HCl, 0.5% glycine |
| 3 | 2.2% arginine-HCl, 0.75% glycine |
| 4 | 3% mannitol, 1% glycine |
| 5 | 3% mannicol, 1% glycine, 1 mM EDTA |
| 6 | 3% mannitol, 1.5% arginine, 0.005% Tween-80 |
| 7 | 3.3% arginine-HCl |
| 8 | 2% mannitol, 2% sucrose, 1.4% arginine |
| 9 | 4% sucrose, 1.4% glycine, 1mM EDTA |

Several formulations, containing similar excipients in similar ratios, nevertheless, surprisingly, do not maintain clotting activity nearly as well. For example, 2.3 % glycine alone gave only 86%; and 4% sucrose, 2% arginine, both with and without Tween, and with and without EDTA gave 87-89% clotting activity.

Shown for the nine formulations of Table 8 are the results of other stability indicating assays. Specific activity is expressed as U/mg and an acceptable range is 200 to 350 U/mg. SEC-HMW is a measure of high molecular weight aggregates as determined by size-exclusion chromatography; less than 1% is preferred for a *subcutaneous, intradermal*, or *intramuscular* formulation. "C-terminal clips" is a measure of degradation species as determined by reversed phase chromatography; less than 1% is preferred.

**TABLE 9**

| **Sample** | **Recovery of Activity** | **Specific Activity** | **SEC HMW** | **C-Terminal Clips** |
|---|---|---|---|---|
| 1 | ≥100% | 262 | 0.24% | 0.31% |
| 2 | ≥100% | 256 | 0.25% | 0.28% |
| 3 | ≥100% | 255 | 0.27% | 0.28% |
| 4 | ≥100% | 262 | 0.26% | 0.33% |
| 5 | ≥100% | 272 | 0.23% | 0.38% |
| 6 | ≥100% | 263 | 0.22% | 0.28% |
| 7 | ≥100% | 258 | 0.24% | 0.19% |
| 8 | ≥100% | 251 | 0.20% | 0.33% |
| 9 | ≥100% | 251 | 0.20% | 0.31% |

Based on the preferred formulations set forth in Tables 8 and 9, two more preferred formulations include as follows: (both are buffered at pH 6.8 with 15 mM citrate and are isotonic), 3% mannitol, 1.5% arginine-HCl; and 3.3% arginine-HCl.

### Example 3: Effects of Freeze/Thaw Cycle

Ideally, a similar formulation is utilized for bulk protein as is used for the finished dosage form. This demands that the same formulation that stabilizes factor IX from long-term storage stresses also be appropriate for stabilizing factor IX from the stresses normally encountered by bulk protein, such as freezing and thawing.

Samples are prepared in the formulations set forth in Table 10 below, at a protein concentration of ∼2 mg/mL (500 U/mL) and an osmolality of 300 ± 50 milliosmolal. All include 10 mM sodium citrate, pH 6.8, and all are prepared both with and without 0.005% Tween-80 (polysorbate).

**Table 10**

| **Sample Formulations** | |
|---|---|
| A. | 2.5% arginine-HCl, 2.2% sucrose |
| B. | 1.8% glycine, 2% sucrose |
| C. | 1.8% arginine-HCl, 2.4% mannitol |
| D. | 2.2% glycine. 0.2% mannitol |
| E. | 2.7% arginine-HCl, 0.8% mannicol |
| F. | 2% arginine-HCl, 2% sucrose, 0.9% mannitol |
| G. | 1.8% arginine-HCl, 2% mannitol, 0.8% sucrose |

Samples of factor IX in each formulation were subjected to five freeze-thaw cycles to determine susceptibility to freezing-induced denaturation, which can result in formation of protein aggregates. A series of freeze-thaw cycles is a useful indication of a protein's susceptibility to increased aggregate formation as may be observed during freezing and long-term storage. Samples are assayed for the amount of HMW present. Samples with and without Tween-80 (0.005%) have minimal aggregation (less than 0.15% HMW increase).

Based on all the data herein, the following two formulations (expressed as ranges of components) are also preferred.

| **Formulation 1:** | | |
|---|---|---|
| sodium citrate | 0.0075 M to 0.04 M | 0.19% to 1% w/v |
| arginine (-HCl) | 0.13 M to 0.16 M | 2.8% to 3.3% w/v |
| sucrose | 0 to 0.06 M | 0 to 2 % w/v |
| polysorbate-80 | 0 to 0.0000382 M | 0 to 0.005 % w/v |
| factor IX | 600 to 56,000 Units/mL | 0.1 to 160 mg/mL |

| **Formulation 2:** | | |
|---|---|---|
| sodium citrate | 0.0075 M to 0.04 M | 0.19% to 1% w/v |
| arginine (-HCl) | 0.06 M to 0.07 M | 1.3 to 1.5% |
| sucrose | 0 to 0.02 M | 0 to 0.7% |
| mannitol | 0.165 M | 3% |
| polysorbate-80 | 0 to 0.0000382 M | 0 to 0.005% w/v |
| factor IX | 600 to 56,000 Units/mL | 0.1 to 160 mg/mL |

### Example 4: Effect of Long Term Storage at 4°C

Factor IX is formulated at 2 mg/mL (500 U/mL) in 15 mM sodium citrate (0.38%), 0.16 M arginine (3.3%), pH 6.8 and stored for one year at 4°C. The recovery of activity is 95% and the % HMW is 0.32%. Factor IX is formulated at 2 mg/mL in 15 mM sodium citrate, 3% mannitol, 1.5% arginine, pH 6.8 and stored for one year at 4°C. The recovery of activity is 76% and the % HMW was 0.36%. The loss of activity is attributed to deamidation.

Factor IX is formulated at 2 mg/mL in 15 mM sodium citrate, 1% = 29 mM sucrose, 3% = 0.14 M arginine HCl and stored for one year at 4°C. The recovery of activity is 86% and % HMW is 0.27.

### Example 5: Effects of High Protein Concentration and of Freeze-Thaw

Factor IX is formulated at 4000 U/mL, 8000 U/mL, 16,000 U/mL and greater than 30,000 U/mL (*i*.*e*., 16 to greater than 120 mg/mL) in 10 mM histidine, 260 mM glycine, 1% sucrose, 0.005% Tween-80, pH 7.0. Factor IX is concentrated by centrifugal concentration in a Centricon-10 and by stir-cell concentration in an Amicon stir cell using a YM-10 membrane. Other methods used for concentrating proteins, especially those using membranes which retain and exclude species based on molecular weight, such as tangential flow filtration, can also be used. In addition, spray-drying can be used with no untoward effects.

Surprisingly, no detectable aggregated protein (HMW as determined by SEC-HPLC) is generated even at these extraordinarily high protein concentrations.

Samples are subsequently frozen and thawed repeatedly and surprisingly still maintain acceptable levels of HMW (≤1%). This is surprising in light of the commercially available plasma-derived factor IX products such as Mononine™ and Alphanine™ (*supra* at page 3, lines 20-29), which frequently contain 10% or greater HMW even though the factor IX concentration is quite low. Such a high % HMW is unacceptable for *subcutaneous*, *intradermal*, or *intramuscular* administration because of the potential for immunogenicity.

Furthermore, when factor IX is formulated in the same formulation as Mononine™ and subjected to repeated cycles of freezing and thawing, significant amounts (-15%) HMW are generated. This data, taken with the data shown in Example 3, demonstrate the surprising and unpredictable effects of formulation on the stability of factor IX.

### Example 6: Use of Highly Concentrated Factor IX

Highly concentrated factor IX is effective when administered *subcutaneously, intradermally* or *intramuscularly.* Utilizing a highly concentrated formulation of factor IX, *i*.*e*., 4,000 U/mL to greater than 56,000 U/mL, a single site, low volume, *subcutaneous* injection is possible as is described below.

Three experimental groups were evaluated using factor IX at a concentration of 4,000 IU/ml in 260 mM glycine, 10 mM histidine, 29 mM (1%) sucrose, and 0.005% polysorbate. In Group I, dogs were given 200 U/kg (0.05 mL/kg) of factor IX *intravenously*. In Group II, dogs were given 200 U/kg (0.05 mL/kg) of factor IX *subcutaneously.* In Group III, dogs were given a factor IX *intravenous* priming dose of 50 U/kg (0.0125 mL/kg) followed 24 hours later by a 200 U/kg (0.05 mL/kg) *subcutaneous* dose. *Intravenous* factor IX produced a 240% factor IX activity (where 100% = pooled human plasma standard) within five minutes of injection which declined to 6.4% by Day 5. *Subcutaneous* factor IX activity was 0.9% at 5 minutes, 10% at three hours and 5.8% on Day 5. The combination of an *intravenous* loading dose followed 24 hours later by a *subcutaneous* dose resulted in a plasma factor IX activity of 25% three hours after the *subcutaneous* dose and a factor IX activity of 9. 1% on Day 5 after the *subcutaneous* injection. The bioavailability of the *subcutaneous* dose was calculated as *43%. Subcutaneous* factor IX produces therapeutic levels of factor IX activity in less than three hours after administration. The combination dose of an *intravenous* with a *subcutaneous* dose provides immediate coagulant protection and improves the efficacy of the *subcutaneous* dose. Also, highly concentrated forms of factor IX can be formulated in the formulations described, *supra*, in Examples 1-3, and effectively used for administration.

While the present invention has been described in terms of specific methods, formulations, and compositions, it is understood that variations and modifications will occur to those skilled in the art upon consideration of the present invention.

Numerous modifications and variations in the invention as described in the above illustrative examples are expected to occur to those skilled in the art and, consequently, only such limitations as appear in the appended claims should be placed thereon. Accordingly, it is intended in the appended claims to cover all such equivalent variations which come within the scope of the invention as claimed.

## Claims

1. A composition comprising highly concentrated factor IX and arginine, a buffering agent, and sucrose or mannitol, wherein the factor IX concentration is about 2 to about 160 mg/mL.

2. The composition of claim 1, wherein said buffering agent is citrate, maleic acid, ammonium acetate, phosphate, histidine, tris or diethanolamine.

3. The composition of any one of claims 1 or 2, further comprising a surfactant or a chelating agent.

4. The composition of any one of claims 1 to 3, wherein said arginine concentration is about 130 to 160 mM, about 130 to 235 mM or about 60 to 70 mM.

5. The composition of claim 4, wherein said arginine concentration is about 160 mM.

6. The composition of any one of claims I to 5, wherein said sucrose concentration is about 3 to 60 mM.

7. The composition of any one of claims 2 to 6, wherein said citrate concentration is about 1 to 40 mM.

8. The composition of claim 7, wherein said citrate concentration is about 15mM.

9. The composition of any one of claims 3 to 8, wherein said surfactant is polysorbate.

10. The composition of claim 9, wherein said polysorbate concentration is about 0.005 to 1%.

11. The composition of claim 3, wherein said arginine concentration is about 130 mM to 160 mM, said sucrose concentration is about 0 to 60 mM, and said surfactant concentration is about 0 to 0.005%.

12. The composition of claim 3, wherein said arginine concentration is about 60 to 70 mM, the sucrose concentration is about 0 to 20 mM, the mannitol concentration is 165 mM and said surfactant is 0 to 0.005%.

13. The composition of claim 3 wherein said arginine concentration is about 160 mM, said sucrose concentration is about 0 mM, said citrate concentration is about 15 mM, and said surfactant concentration is about 0%.

14. The composition of claim 3, wherein said arginine concentration is about 70 mM, said mannitol concentration is about 165 mM, said citrate concentration is about 15 mM, and said surfactant or chelating agent is 0%.

15. The composition of claim 2 comprising about:
2 to 160 mg/mL factor IX,
130 to 235 mM arginine, and
7.5 to 40 mM citrate.

16. The composition of claim 9 comprising about:
2 to 160 mg/mL factor IX,
130 to 235 mM arginine,
0 to 60 mM sucrose,
0 to 0.005% polysorbate, and
7.5 to 40 mM citrate.

17. A composition of claim 2 comprising about:
2 to 160 mg/mL factor IX,
66 to 90 mM arginine,
110 to 165 mM mannitol, and
7.5 to 40 mM citrate.

18. A composition of claim 1 comprising about:
2 to 160 mg/mL factor IX,
70 mM arginine,
165 mM mannitol, and
15 mM citrate.

19. A composition of claim 2 comprising about:
2 to 160 mg/mL factor IX,
160 mM arginine, and
15 mM citrate.

20. Use of a composition of any one of claims 1 to 19 for the preparation of a pharmaceutical composition for increasing factor IX concentration.

21. The use of claim 20 wherein said pharmaceutical composition is designed to be administered both intravenously and subcutaneously.

## Patentansprüche

1. Zusammensetzung, welche hochgradig konzentrierten Faktor IX und Arginin, eine Puffersubstanz und Saccharose oder Mannitol umfasst, worin die Faktor IX Konzentration etwa 2 bis etwa 160 mg/ml beträgt.

2. Zusammensetzung nach Anspruch 1, worin besagte Puffersubstanz Citrat, Maleinsäure, Ammoniumacetat, Phosphat, Histidin, Tris- oder Diethanolamin ist.

3. Zusammensetzung nach einem der Ansprüche 1 oder 2, welche ferner einen Surfactanten oder einen Chelatbildner umfasst.

4. Zusammensetzung nach einem der Ansprüche 1 bis 3, worin besagte Argininkonzentration etwa 130 bis 160 mM, etwa 130 bis 235 mM oder etwa 60 bis 70 mM beträgt.

5. Zusammensetzung nach Anspruch 4, worin besagte Argininkonzentration etwa 160 mM beträgt.

6. Zusammensetzung nach einem der Ansprüche 1 bis 5, worin besagte Saccharosekonzentration etwa 3 bis 60 mM beträgt.

7. Zusammensetzung nach einem der Ansprüche 2 bis 6, worin besagte Citratkonzentration etwa 1 bis 40 mM beträgt.

8. Zusammensetzung nach Anspruch 7, worin besagte Citratkonzentration etwa 15 mM beträgt.

9. Zusammensetzung nach einem der Ansprüche 3 bis 8, worin besagter Surfactant Polysorbat ist.

10. Zusammensetzung nach Anspruch 9, worin besagte Polysorbatkonzentration etwa 0,005 bis 1% beträgt.

11. Zusammensetzung nach Anspruch 3, worin besagte Argininkonzentration etwa 130 mM bis 160 mM beträgt. besagte Saccharosekonzentration etwa 0 bis 60 mM beträgt und besagte Surfactantkonzentration etwa 0 bis 0,005% beträgt.

12. Zusammensetzung nach Anspruch 3, worin besagte Argininkonzentration etwa 60 bis 70 mM beträgt, die Saccharosekonzentration etwa 0 bis 20 mM beträgt, die Mannitolkonzentration 165 mM beträgt und besagter Surfactant 0 bis 0,005% beträgt.

13. Zusammensetzung nach Anspruch 3, worin besagte Argininkonzentration etwa 160 mM beträgt, besagte Saccharosekonzentration etwa 0 mM beträgt, besagte Citratkonzentration etwa 15 mM beträgt und besagte Surfactantkonzentration etwa 0% beträgt.

14. Zusammensetzung nach Anspruch 3, worin besagte Argininkonzentration etwa 70 mM beträgt, besagte Mannitolkonzentration etwa 165 mM beträgt, besagte Citratkonzentration etwa 15 mM beträgt und besagter Surfactant oder Chelatbildner 0% beträgt.

15. Zusammensetzung nach Anspruch 2, welche etwa umfasst:
2 bis 160 mg/ml Faktor IX,
130 bis 235 mM Arginin und
7,5 bis 40 mM Citrat.

16. Zusammensetzung nach Anspruch 9, welche etwa umfasst:
2 bis 160 mg/ml Faktor IX,
130 bis 235 mM Arginin,
0 bis 60 mM Saccharose
0 bis 0,005% Polysorbat und
7,5 bis 40 mM Citrat.

17. Zusammensetzung nach Anspruch 2, welche etwa umfasst:
2 bis 160 mg/ml Faktor IX,
66 bis 90 mM Arginin,
110 bis 165 mM Mannitol und
7,5 bis 40 mM Citrat.

18. Zusammensetzung nach Anspruch 1, welche etwa umfasst:
2 bis 160 mg/ml Faktor IX,
70 mM Arginin,
165 mM Mannitol und
15 mM Citrat.

19. Zusammensetzung nach Anspruch 2, welche etwa umfasst:
2 bis 160 mg/ml Faktor IX,
160 mM Arginin und
15 mM Citrat.

20. Verwendung einer Zusammensetzung nach einem der Ansprüche 1 bis 19 zur Herstellung einer pharmazeutischen Zusammensetzung zum Erhöhen von Faktor IX Konzentration.

21. Verwendung nach Anspruch 20, wobei besagte pharmazeutische Zusammensetzung konstruiert wird, um sowohl intravenös, als auch subkutan verabreicht zu werden.

## Revendications

1. Composition comprenant un facteur IX hautement concentré et de l'arginine, un agent tampon et du saccharose ou du mannitol, dans laquelle la concentration en facteur IX est d'environ 2 à environ 160 mg/ml.

2. Composition suivant la revendication 1, dans laquelle ledit agent tampon est du citrate, de l'acide maléique, de l'acétate d'ammonium, du phosphate, de l'histidine, de la triséthanolamine ou diéthanolamine.

3. Composition suivant l'une quelconque des revendications 1 ou 2, comprenant en outre un tensioactif ou un agent chélatant.

4. Composition suivant l'une quelconque des revendications 1 à 3, dans laquelle ladite concentration en arginine est d'environ 130 à 160 mM, d'environ 130 à 235 mM ou d'environ 60 à 70 mM.

5. Composition suivant la revendication 4, dans laquelle ladite concentration en arginine est d'environ 160 mM.

6. Composition suivant l'une quelconque des revendications 1 à 5, dans laquelle ladite concentration en saccharose est d'environ 3 à 60 mM.

7. Composition suivant l'une quelconque des revendications 2 à 6, dans laquelle ladite concentration en citrate est d'environ 1 à 40 mM.

8. Composition suivant la revendication 7, dans laquelle ladite concentration en citrate est d'environ 15 mM.

9. Composition suivant l'une quelconque des revendications 3 à 8, dans laquelle ledit tensioactif est du polysorbate.

10. Composition suivant la revendication 9, dans laquelle ladite concentration en polysorbate est d'environ 0,005 à 1%.

11. Composition suivant la revendication 3, dans laquelle ladite concentration en arginine est d'environ 130 mM à 160 mM, ladite concentration en saccharose est d'environ 0 à 60 mM, et ladite concentration en tensioactif est d'environ 0 à 0,005%.

12. Composition suivant la revendication 3, dans laquelle ladite concentration en arginine est d'environ 60 à 70 mM, la concentration en saccharose est d'environ 0 à 20 mM, la concentration en mannitol est de 165 mM et ladite concentration en tensioactif est d'environ 0 à 0,005%.

13. Composition suivant la revendication 3, dans laquelle ladite concentration en arginine est d'environ 160 mM, ladite concentration en saccharose est d'environ 0 mM, ladite concentration en citrate est d'environ 15 mM, et ladite concentration en tensioactif est d'environ 0%.

14. Composition suivant la revendication 3, dans laquelle ladite concentration en arginine est d'environ 70 mM, ladite concentration en mannitol est d'environ 165 mM, ladite concentration en citrate est d'environ 15 mM, et ledit tensioactif ou agent chélatant est présent à 0%.

15. Composition suivant la revendication 2 comprenant environ :
2 à 160 mg/ml de facteur IX,
de l'arginine 130 à 235 mM, et
du citrate 7,5 à 40 mM.

16. Composition suivant la revendication 9 comprenant environ :
2 à 160 mg/ml de facteur IX,
de l'arginine 130 à 235 mM,
du saccharose 0 à 60 mM,
du polysorbate à de 0 à 0,005%, et
du citrate à 7,5 à 40 mM.

17. Composition suivant la revendication 2 comprenant environ :
2 à 160 mg/ml de facteur IX,
de l'arginine 66 à 90 mM,
du mannitol 110 à 165 mM, et
du citrate à 7,5 à 40 mM.

18. Composition suivant la revendication 1 comprenant environ :
2 à 160 mg/ml de facteur IX,
de l'arginine 70 mM,
du mannitol 165 mM, et
du citrate à 15 mM.

19. Composition suivant la revendication 2 comprenant environ :
2 à 160 mg/ml de facteur IX,
de l'arginine 160 mM, et
du citrate à 15 mM.

20. Utilisation d'une composition suivant l'une quelconque des revendications 1 à 19, pour la préparation d'une composition pharmaceutique pour augmenter la concentration en facteur IX.

21. Utilisation suivant la revendication 20, dans laquelle ladite composition pharmaceutique est conçue pour être administrée à la fois par voie intraveineuse et sous-cutanée.
